# EUROPEAN PATENT APPLICATION

(11) **EP 0 637 591 A2**
(43) Date of publication of application: **08.02.1995**
(21) Application number: 93119118.3
(22) Date of filing: 26.11.1993
(51) Int. Cl.: C12N 15/70, C12N 1/21, C12P 21/02, C07K 14/415

(54) **A novel expression vector for phytolacca antiviral protein**

(30) Priority: 02.07.1993 KR 9312360
(71) Applicant: JINRO LIMITED, Seoul (KR)
(72) Inventor: Moon, Young-Ho, Kuri, Kyunggi-Do (KR); Jeon, Hong-Seob, Seoul (KR); Choi, Kyu-Whan, Seoul (KR); Lee, Kwan-Ho, Pundang-Ku, Seongnam, Kyunggi-Do (KR); Kim, Man-Keun, Youngdeungpo-Ku, Seoul (KR)
(74) Representative: TER MEER - MÜLLER - STEINMEISTER & PARTNER

(57) **Abstract**

The present invention provides a novel expression vector for Phytolacca antiviral protein isolated from Phytolacca americana L. and a microorganism transformed with said PAP expression vector. In accordance with the present invention, biologically active PAP can be produced in a massive manner from the microorganism transformed with the expression vector of the invention; and, therefore, the recombinant PAP can be practically applied to various fields, e.g., the molecular studies on PAP and treatment of AIDS employing immunoconjugate.

## Description

### Field of the Invention

The present invention relates to a novel expression vector for antiviral protein, more specifically, a recombinant expression vector for Phytolacca antiviral protein isolated from Phytolacca americana L. and a microorganism transformed therewith.

### Background of the Invention

Studies on the antiviral proteins from many different plant species, have been carried out, starting from the discovery of pokeweed antiviral protein(or Phytolacca antiviral protein, hereinafter referred to as "PAP") isolated from crude extract of Phytolacca americana L.[see: Irvin, J. D., Arch. Biochem. Biophys., 169: 522-528(1975)]. In addition to the PAP, a few of antiviral proteins have been isolated from several plants, e.g., Ricin(from Ricinus communis)[see: Halling, K. C. et al., Nucleic Acid Res., 13: 8019-8033(1985)], Mirabilis antiviral protein("MAP", from Mirabilis jalapa L.)[see: Kataoka, J. et al., J. Biol. Chem., 266: 8426-8430(1991)] and α-trichosanthin(from Trichosanthes kirilowii) [see: Zhang, X. et al., Nature, 321: 477-478(1986)]. Said antiviral proteins have been reported to be ribosome inactivating proteins("RIPs") having RNA N-glycosidase activities[see: Endo, Y. et al., J. Biol. Chem., 263: 8735-8739(1988)].

In general, PAP from Phytolacca americana L. is classified as PAP-I, PAP-II and PAP-S that appear in spring leaves, summer leaves and seeds, respectively; and it is reported that antiserum reactions of these PAPs are different one another[see: Irvin, J. D. et al., Arch. Biochem. Biophys., 200: 418-425(1980)]. Further, it has been known that ribosome of Phytolacca americana L. is depurinated by RNA N-glycosidase activity of PAP. On the other hand, immunoconjugate of PAP with CD4 or CD19 has been reported to inhibit the replication of human immunodificiency virus type 1[see: Jansen, B. et al., Cancer Res., 52: 406-412(1992); Kim Y. W. et al., J. Immunol., 144: 1257-1262(1990); Myers, D. E. et al., J. Immunol. Methods, 136: 221-238(1991)]; in this connection, said PAPs have been supposed to be applicable to the treatment of AIDS. Accordingly, studies on the PAPs in a view of molecular biology have been actively carried out, including the nucleotide sequence analysis of cDNA of PAP; precise mechanism of PAP's biological activity; construction of transgenic plant; and, application to the immunoconjugate preparation.

Under the circumstance, the present inventors firstly developed a transgenic plant expressing PAP, and patent applications covering said expression vector are pending under the title of "Expression vector for Phytolacca antiviral protein and process for preparing transgenic plant transformed thereof"(USSN 08/049,075; EP appln. No. 93 110 445.9; JP appln. No. 5-128222). However, it is clear that the expression vector of prior art is simply designed for the purpose of transformation of plants to confer viral resistance, in light of employing CaMV 35S promoter which is generally used for the expression of plant gene.

Accordingly, the expression of PAP gene has been restrictive in light of host organism, i.e, it has been only expressed in plant; and, therefore, needs have continued to exist for the development of a practical expression vector, which produces PAP in a versatile microorganism with high expression level.

### Summary of the Invention

In accordance with the present invention, the present inventors, for the first time, developed a recombinant vector which expresses the PAP gene in a microorganism transformed therewith.

A primary object of the present invention is, therefore, to provide a novel recombinant expression vector containing PAP gene isolated from cDNA library of the Phytolacca americana L., which can be expressed in microorganisms with high yield.

Another object of the present invention is to provide a microorganism transformed therewith which expresses PAP gene in large quantities.

### Brief Description of Drawings

The above and the other objects and features of the present invention will become apparent from following descriptions given in conjunction with the accompanying drawings, in which:
- Figure 1: is full nucleotide sequence of PAP gene isolated from cDNA library of Phytolacca americana L.;
- Figure 2: is a scheme depicting stepwise construction strategy for expression vector pMJ12 of the invention;
- Figure 3: is a photograph showing electrophoresis pattern of expression vector pMJ12;
- Figure 4: is a graph showing growth pattern of E. coli HB101 harboring pMJ12;
- Figure 5: is a photograph showing SDS-PAGE pattern of PAP purified from E. coli HB101 harboring pMJ12;
- Figure 6: is a photograph showing the Western blot analysis of PAP purified from E. coli HB101 harboring pMJ12; and,
- Figure 7: is a photograph showing SDS-PAGE pattern of in vitro translation employing rabbit reticulocyte lysate system.

### Detailed Description of the Invention

The present inventors developed a recombinant expression vector pMJ12 containing PAP gene isolated from cDNA library of Phytolacca americana L., which produces recombinant PAP in microorganism transformed therewith.

To isolate PAP gene, the inventors purified total cellular mRNA from leaves of Phytolacca americana L. obtained in Korea and construction of cDNA library thereof is followed. PAP gene is selected by immunoscreening method employing anti-PAP antibody; and, deletion mutant is prepared from the isolated PAP gene using Erase-a-Base system(Promega, U.S.A.). DNA sequence of a cDNA clone containing PAP gene is determined by Sanger's dideoxy chain termination method[see: Sanger, F., Science, 214: 1205-1210(1981)].

For the expression of isolated PAP gene in microorganisms, a commercially available FLAG™ vector(International Biotechnologies Inc., U.S.A.) is employed. Since isolated PAP gene has signal peptide, coding region of mature PAP is amplified by Thermal Cycler™, which employes synthesized N-terminal primer and C-terminal primer. DNA thus amplified is electroeluted, digested with HindIII, ligated into FLAG™ and recombinant expression vector pMJ12 is constructed. Said pMJ12 is deposited with the Korean Collection of Culture and Microorganism(KCCM), an International Depository Authority(IDA) on June 30, 1993 as deposition No. KCCM 10037; and claimed in the invention.

pMJ12 thus constructed is transformed into competent E. coli HB101 and colonies transformed with pMJ12 are selected. Then, recombinant PAP is induced for 6hrs, by culturing said colony on LB broth media containing IPTG(isopropyl-β-D-thiogalactoside) and ampicillin. After recombinant PAP induction, cells thus cultured are harvested, washed with phosphate buffered saline(PBS: 0.01M NaH₂PO₄, 0.15M NaCl, pH 7.4) solution two times; and lysis of cell pellet is carried out by freezing in dry ice-methanol bath and thawing at 37°C, repeatedly. Then, centrifugation of cell lysate is followed and supernatant thereof is collected. 10µl of supernatant thus obtained is fractionated by SDS-PAGE, stained with Coomassie brilliant blue R, and production of recombinant PAP is determined by Western blot analysis.

For the purification of recombinant PAP, said supernatant is loaded on anti-FLAG M1 affinity column and eluted with the PBS solution containing 1.0mM CaCl₂. Biological activity of recombinant PAP is determined by in vitro translation method.

The present invention is further illustrated in the following examples, which should not be taken to limit the scope of the invention.

### Example 1: Isolation of PAP gene from cDNA library

PAP was isolated in accordance with Irvin's method[see: J.D. Irvin, et al., Arch. Biochem. Biophys., 169: 522-528(1975)]. 480µg of PAP was dissolved in PBS solution and combined with Freund's complete adjuvant with a ratio of 1:1(v/v), and mixture thereof was administrated to a rabbit(~3Kg of body weight) intramuscularly. After 3 weeks, antibody formation was detected in a small amount of blood collected from the rabbit, and 750µg of PAP was combined with Freund's incomplete adjuvant for boosting with a ratio of 1:1(v/v). Plasma fraction was fractionated from blood by centrifugation; and Protein-A agarose column chromatography was employed to isolate antiplasma. Immunodiffusion assay and electrophoresis technique were employed to determine antibody formation and homogeniety of isolated anti-PAP antibody, respectively. Purified anti-PAP antibody was stored at -70°C and employed to isolate PAP gene from cDNA library.

To isolate mRNA from the leaves of Phytolacca americana, the leaf tissue was homogenated using liquid nitrogen and, to the homogenate thus prepared was added buffer solution for total RNA isolation. Centrifugation was carried out to give supernatant; and total cellular RNA was isolated from the supernatant by LiCl sedimentation. Then, mRNA was isolated from the total RNA using oligo(dT) cellulose column chromatography, and isolated mRNA was employed for cDNA synthesis.

1st strand cDNA was synthesized from template mRNA employing M-MuLV reverse transcriptase; and, 2nd strand cDNA synthesis by E. coli DNA polymerase was followed. Synthesized cDNA was linked to EcoRI adaptor and subjected to Sephacryl S-400 spun column to fractionate DNA fragments in accordance with molecular size of cDNA. Fractionated cDNA was ligated into Uni-Zap XR vector(Stratagene Co., U.K.), and in vitro packaging using packaging extract was followed. Then, immunoscreening employing anti-PAP antibody was carried out to isolate PAP gene from cDNA library thus prepared. E. coli SURE was infected with phage to form plaques of 2 x 10⁴ pfu. Said bacteria was incubated at 37°C for 15min, and further cultured at 42°C for 3.5 hours after plating with 3ml of top agarose. Then, plate was covered with Hybond-N+(Amersham) and incubated for 5 hours. After incubation, Hybond-N+ was blocked with bovine serum albumin and treated with 5mg/ml of anti-PAP antibody. After the removal of free anti-PAP antibody which was not bound, peroxidase conjugated 2nd antibody was reacted with anti-PAP antibody; and, antigen-antibody complexes thus formed were detected by chloronaphthol treatment.

2nd immunoscreening procedure was followed with 15 clones obtained from the 1st immunoscreening method, in a similar fashion as aboves with the exception of plaque number 5 x 10³ pfu. 3rd immunoscreening procedure was performed with the clones obtained from the 2nd immunoscreening, and 8 plaques isolated were subjected to the following experiment.

To transfer phagemids of 8 recombinant Uni-Zap XR phages obtained from the 3rd immunoscreening method, in vivo excision technique employing R408 helper phage was carried out. Phagemids were isolated from 4 colonies by alkali denaturation method[see: Maniatis et al., Molecular Cloning: A Laboratory Manual, pp 368-369, Cold Spring Harbor Laboratory(1982)], and colonies harboring PAP cDNA insert were screened by restriction enzyme excision method.

Of the clones thus screened, plasmids from 2 clones were isolated, and PAP cDNA insert was sequenced by dideoxy chain termination method. To determine full nucleotide sequence of PAP gene, DNA was purified from microorganisms harboring PAP gene. DNA thus purified was digested with SacII and BamHI and deletion was made by intermittent ExoIII excision reaction using Erase-a-Base system (Promega, U.S.A.). Deleted DNAs were ligated each other by T₄ DNA ligase and the resultant was transformed into competent XL1-BLUE cell prepared by the treatment of CaCl₂ solution. Deletion mutants fractionated by molecular size were employed to determine DNA sequence.

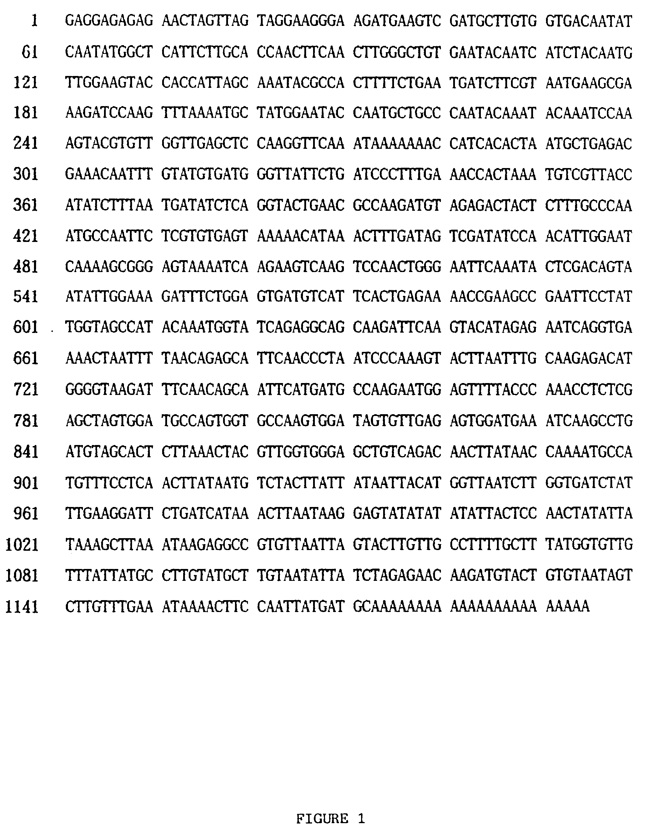

After single strand preparation by alkali denaturation method, full DNA sequence of PAP gene was determined by SEQUENASE VERSION2.0(United States Biochemical, U.S.A.) employing primer such as T₇ promoter primer or universal reverse primer. As disclosed in Figure 1, PAP cDNA comprises 1195bp of one open reading frame; and cDNA insert of PAP codes 313 amino acid residues, 22 residues of which functions as signal peptide.

### Example 2: Preparation of expression vector pMJ12

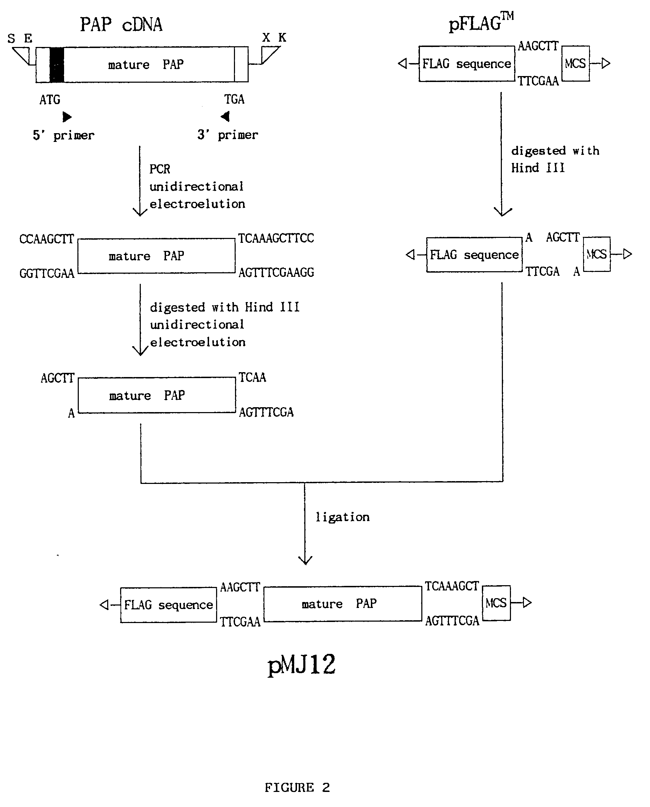

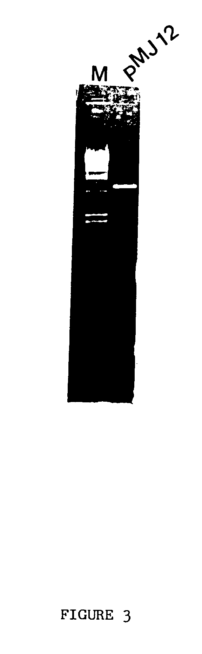

For the expression of PAP gene in E. coli HB101, commercially available FLAG™ vector(International Biotechnologies Inc., U.S.A.) was employed. Primers such as 5' -CCAAGCTTGTGAATACAATCAAC-3' and 5' -GGAAGCTTTGATCAGAATCCTTCAAA-3' synthesized by DNA Synthesizer(Applied Biosystems Inc., U.S.A.), were employed as N-terminal primer and C-terminal primer, respectively; and mature PAP gene was amplified by polymerase chain reaction using Vent™ DNA polymerase(New England Biolab., U.S.A.). In this connection, denaturation(95°C, 30sec), annealing(55°C, 30sec) and extension(72°C, 30sec) were carried out for 30 cycles by DNA Thermal Cycler (Cetus/Perkin-Elmer, U.S.A.). PAP gene thus amplified was cleaved with HindIII, and expression vector of the invention was constructed by ligating HindIII-cleaved PAP gene with HindIII-cleaved FLAG™ vector with the aid of T₄ DNA ligase. Expression vector thus constructed was named pMJ12 and claimed in the invention. The process for stepwise construction of pMJ12 is depicted in Figure 2. In Figure 2, S, E, X and K are employed to mean SacI, EcoRI, XhoI and KpnI restriction enzymes, respectively; and, ( 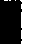 ) indicates signal peptide. pMJ12 thus constructed was transformed into competent E. coli HB101 prepared by the treatment of CaCl₂ solution; and, transformant harboring pMJ12 was selected from the colonies cultured on LB media containing 50µg/ml ampicilin, based on plasmid DNA isolation technique employing alkaline lysis method. E. coli HB101 thus transformed was claimed in the invention and deposited with the Korean Culture Center of Microorganisms(KCCM), an International Depository Authority(IDA) on June 30, 1993 as deposition No. KCCM 10037. Figure 3 is a photograph showing 0.8% agarose gel electrophoresis pattern of pMJ12 digested with HindIII. In Figure 3, M is molecular marker lane, i.e., λDNA fragments digested with HindIII, and pMJ12 lane shows expression vector pMJ12 of the present invention. As shown in Figure 3, molecular size of pMJ12 vector was determined to be about 0.8kb.

### Example 3: Growth inhibition of microorganism transformed with pMJ12

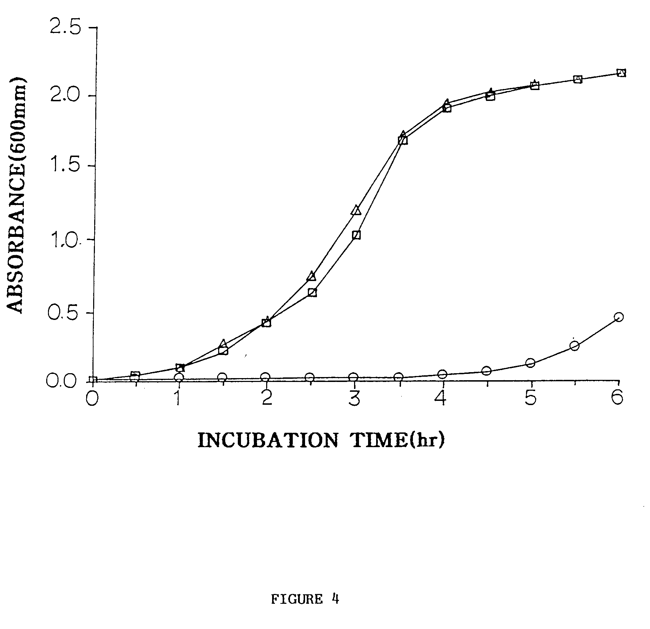

Ricin, a kind of ribosome inactivating protein(RIP), has been reported to provide no effect on the growth of host microorganism, while expression of MAP gene inhibit growth of its transformant. Under the circumstance, whether recombinant PAP produced from the expression vector pMJ12 inhibits growth of transformant or not, was studied through the investigation of cell growth pattern of host organism. Non-transformed E. coli HB101 and E. coli HB101 transformed with pFLAG or pMJ12 were inoculated on LB liquid media containing 50µg/ml ampicilin, and incubated overnight. Then, each culture of the same cell concentration was pipetted, inoculated on LB liquid media containing 0.7mM IPTG(isopropyl-β-D-thiogalactoside) and cultured on shaking incubator at 37°C, 200rpm; and, cell concentration of each culture was determined as absorbance at 600nm. As clearly illustrated in Figure 4, the growth of HB101(o-o) harboring pMJ12 which produce recombinant PAP was inhibited remarkably, while those of non-transformed HB101 (△-△) and HB101(□-□) transformed with pFLAG were normal. Accordingly, it was determined that recombinant PAP inhibits the growth of E. coli HB101 transformed with pMJ12 by virtue of PAP's RNA N-glycosidase activity.

### Example 4: Expression of recombinant PAP from E. coli HB101 transformed with vector pMJ12

E. coli HB101(KCCM 10037) was cultured on 50ml of LB medium containing 50µg/ml ampicilin; and recombinant PAP was induced by the addition of IPTG(0.75mM) when OD₆₀₀ of the culture was reached to the level of 1.0. After PAP induction for 6hrs, cells thus cultured were harvested by centrifugation, washed 2 times with phosphate buffered saline(PBS: 0.01M NaH₂PO₄, 0.15M NaCl, pH 7.4); and subjected to freezing in dry ice-methanol bath and thawing at 37°C. Then, cell lysate was emulsified with said buffer solution(pH 8.4) containing 0.25mg/ml lysozyme; and freezing in dry ice-methanol bath and thawing process was repeated 3 times. Said solution was shaked at an interval of 10 min, kept in 37°C for 30min and centrifuged at 25,000 x g for 45min, at 4°C. 10µl of supernatant thus obtained was analyzed by 15% SDS-PAGE, stained with Coomassie brilliant blue R, and destained with destaining solution; and production of recombinant PAP was determined by Western blot analysis in accordance with the Example 6.

### Example 5: Isolation of recombinant PAP from E. coli HB101 transformed with vector pMJ12

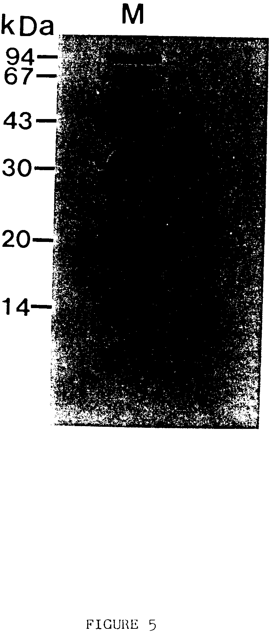

Recombinant PAP produced from E. coli HB101(KCCM 10037) was isolated under the temperature of 4°C, in accordance with the following procedures: To the total protein fractionated in Example 4 was added 1M CaCl₂ solution to the final concentration of 1.0mM; and said solution was loaded on anti-FLAG M1 affinity gel column, after washing with 5ml glycine-HCl(pH 3.0) and PBS solution 3 times. Then, said column was washed with 12ml PBS/Ca solution(PBS solution containing 1.0mM CaCl₂ solution) 3 times. 500µl of PBS/EDTA solution(PBS solution containing 2.0mM EDTA) was kept for 30min in the column to which recombinant PAP was bound; and eluted with 500µl PBS/EDTA solution at an interval of 10min. Amount and purity of recombinant PAP thus isolated were determined by Bradford's method[see: Bradford, Anal. Biochem., 72: 248(1976); Anal. Biochem., 86: 142(1978)] and SDS-PAGE analysis, respectively. The expression level of said recombinant PAP was determined to be 40mg per 1L of culture, which is relatively high expression, compared with other RIPs. Figure 5 is a photograph showing SDS-PAGE pattern of purified recombinant PAP. In Figure 5, M shows low-molecular size marker(Pharmacia, U.S.A.) lane; recombinant PAP is shown on the right hand.

### Example 6: Determination of recombinant PAP from E. coli HB101 transformed with vector pMJ12

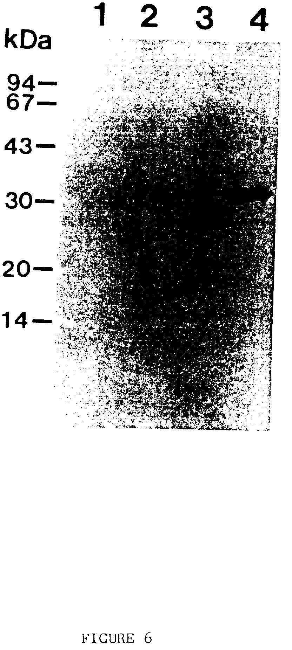

Total proteins of non-transformed E. coli HB101 and E. coli HB101 transformed with vector pFLAG or pMJ12 were isolated in accordance with the Example 4, respectively; and, Western blot analysis was followed. Protein fractionated by SDS-PAGE, was transferred to Hybond-C extra (Amersham, U.K.); and, blocking was made with PBS solution containing 0.1% Tween 20 and 2% BSA. Said protein was washed with PBS solution(containing 0.1% Tween 20) 2 times for 5min; and, treated with 2µg/ml of anti-PAP antibody at room temperature for 1hr. Then, the resultant was washed with said buffer solution 2 times, treated with rabbit peroxidase-conjugated second antibody at room temperature for 1hr, and staining with 4-chloro-1-naphthol was followed. As shown in the result of Western blot analysis of Figure 6, only one band was determined on E. coli HB101 transformed with pMJ12, and no band was detected on non-transformed HB101(lane 1) and HB101 transformed with pFLAG(lane 2).

### Example 7: Activity determination of recombinant PAP

To determine the activity of purified recombinant PAP which inhibits protein synthesis, in vitro translation employing rabbit reticulocyte lysate system(Promega, U.S.A.) was carried out. Recombinant PAP isolated in accordance with the Example 5 and dialysed against deionized water by Spectra/Por 2 membrane(Spectrum, U.S.A.) system, was employed for the activity determination. The reaction mixtures for in vitro translation were disclosed in Table 1.

**Table 1**

| Reaction mixtures for in vitro translation | | |
|---|---|---|
| Experimental group | Control | PAP |
| Reaction mixture | 35µl rabbit reticulocyte lysate | 3*5*µl rabbit reticulocyte lysate |
| | 1mM amino acids (methionine free) | 1mM amino acids (methionine free) |
| | 1µl ³⁵S-methionine (10mCi/ml) | 1µl ³⁵S-methionine (10mCi/ml) |
| | 1µl RNasin (40U/µl) | 1µl RNasin (40U/µl) |
| | 2µl luciferase RNA (0.5µ*g*/µl) | 2µl luciferase RNA (0.5µ*g*/µl) |
| | 11µl Water | 11µl recombinant PIP (80pmol) |

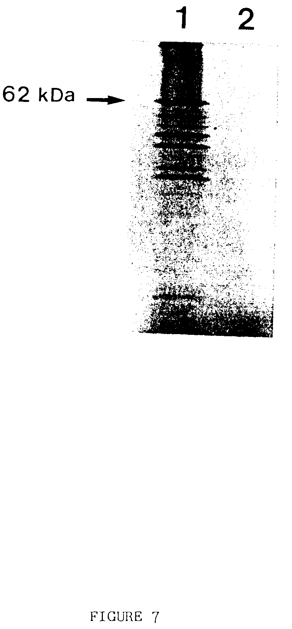

Each experimental group was incubated at 30°C for 90min. Proteins thus synthesized were fractionated by 15% SDS-PAGE, dried with gel dryer and determined by radioautography. Figure 7 is a photograph showing the results of SDS-PAGE after in vitro translation experiment. As clearly illustrated in Figure 7, protein synthesis of luciferase(62KD) is appeared in control group(lane 1), while no protein is detected in recombinant PAP(lane 2) group.

As cleary illustrated and demonstrated as aboves, the present invention provides a novel expression vector for PAP, a microorganism transformed with said PAP expression vector. In accordance with the present invention, biologically active PAP could be produced in a massive manner from the microorganism transformed with expression vector of the invention; and, therefore, the recombinant PAP would be applied to various fields, e.g., the molecular studies on PAP and treatment of AIDS employing immunoconjugate.

## Claims

1. A recombinant expression vector pMJ12(KCCM 10037) which produces Phytolacca antiviral protein in microorganism.

2. E. coli HB101(KCCM 10037) transformed with the recombinant expression vector of claim 1, which produces Phytolacca antiviral protein.

3. A process for preparing Phytolacca antiviral protein, which comprises step of culturing the transformed E. coli HB101(KCCM 10037).

4. Phytolacca antiviral protein produced by the process of claim 3.
